Europäisches Patentamt

European Patent Office

Office européen des brevets

(19)

(11) Numéro de publication : **0 190 949**
**B1**

(12) **FASCICULE DE BREVET EUROPÉEN**

(45) Date de publication du fascicule du brevet :
05.04.89

(21) Numéro de dépôt : 86400006.2

(22) Date de dépôt : 03.01.86

(51) Int. Cl.⁴ : **B 01 J 29/28, C 07 C 41/09**

(54) **Obtention d'une offrétite synthétique riche en silice, application à la production d'hydrocarbures riches en oléfines légères par transformation du méthanol et/ou de diméthyléther.**

(30) Priorité : 09.01.85 FR 8500331

(43) Date de publication de la demande :
13.08.86 Bulletin 86/33

(45) Mention de la délivrance du brevet :
05.04.89 Bulletin 89/14

(84) Etats contractants désignés :
BE DE GB IT NL

(56) Documents cités :
DE–A– 2 420 850
FR–A– 2 492 838
FR–A– 2 569 678
US–A– 3 591 488
US–A– 3 758 539
US–A– 3 925 191
US–A– 4 339 353
CHEMICAL ABSTRACTS, vol. 81, no. 4, 29 juillet 1974,
page 243, résumé no. 16974d, Columbus, Ohio, US;
R.M. BARRER et al.: "Optimization and modification
of offretite sorbents", & SEPAR. SCI. 1974, 9(3), 195-
209

(73) Titulaire : **INSTITUT FRANCAIS DU PETROLE**
**4, Avenue de Bois-Préau**
**F-92502 Rueil-Malmaison (FR)**

(72) Inventeur : **Ferre, Gilbert**
**"Le Trident" 72, Rond Point du Pont de Sèvres**
**F-92100 Boulogne sur Seine (FR)**
Inventeur : **Dufresne, Pierre**
**67, rue Georges Sand**
**F-92500 Rueil Malmaison (FR)**
Inventeur : **Marcilly, Christian**
**91 ter rue Condorcet**
**F-78800 Houilles (FR)**

**Description**

La présente invention concerne des offrétites synthétiques enrichies en silice et l'utilisation de certaines de ces offrétites modifiées à la production d'hydrocarbures et tout particulièrement d'oléfines légères comprenant 2 à 4 atomes de carbone, par molécule, à partir de méthanol, de diméthyléther ou de leur mélange.

Le méthanol peut être préparé à partir de diverses sources carbonées d'origine non pétrolière, telles que le gaz naturel (méthane essentiellement) et le charbon en passant par le gaz de synthèse CO + H$_2$, ou telle que les végétaux par transformation de cellulose. Le méthanol pourrait ainsi constituer dans les années à venir une matière de base essentielle de la chimie pour la fabrication des grands intermédiaires de synthèse, ne serait-ce que par la facilité de son transport et de sa manipulation.

De nombreux travaux ont été consacrés à la conversion du méthanol en hydrocarbures. Les catalyseurs les plus fréquemment utilisés et les plus performants sont des aluminosilicates cristallins du type zéolithe. Pour être actives et sélectives, les zéolithes issues de la synthèse hydrothermale doivent être au préalable débarrassées des cations alcalins que renferment généralement leurs structures. Elles développent alors des acidités très élevées qui leur permettent d'atteindre des activités très grandes dans la transformation du méthanol en hydrocarbures. Un inconvénient majeur de la plupart de ces zéolithes est leur désactivation très rapide en quelques minutes ou dizaines de minutes par formation de dépôts carbonés. Parmi les premiers et principaux brevets mentionnant l'utilisation de zéolithes, on peut mentionner les brevets américains suivants : US-A-3 036 134 de MATTOX et US-A-3 529 033 de FRILETTE et WEISZ.

Depuis l'utilisation des premières zéolithes, des améliorations substantielles ont été apportées notamment grâce aux deux progrès suivants :

1) découverte de nouvelles structures zéolithiques, notamment plus riches en silice, grâce à de nouvelles méthodes de synthèses (sur lesquelles les premiers travaux ont été entrepris au début des années 1960) : c'est le cas des zéolithes de la série ZSM, des zéolithes NU-1, FU-1 etc...

2) mise au point de techniques de modification de la charpente aluminosilicatée de structures zéolithiques déjà connues (ces dernières sont souvent utilisables avec les nouvelles structures) qui permettent de modifier à volonté l'acidité de ces solides : c'est le cas des zéolithes du type faujasite et plus précisément de la zéolithe Y (C. V. McDANIEL et P. K. MAHER in « Molecular Sieves », Society of Chemical Industry, London, 1968, p. 186-195) ; c'est aussi le cas de la mordénite (EBERLY, US-A-3 506 400).

D'une manière générale, il apparaît que les zéolithes les plus intéressantes pour la conversion du méthanol en hydrocarbures, sont celles présentant des rapports molaires SiO$_2$/Al$_2$O$_3$ voisins de, ou supérieurs à 10. Des rapports aussi élevés peuvent être obtenus soit directement à l'issue de la synthèse hydrothermique de la zéolithe, soit en modifiant par des traitements appropriés la structure de zéolithes synthétisées au départ avec des rapports molaires SiO$_2$/Al$_2$O$_3$ plus faibles.

Les zéolithes acides les plus fréquemment utilisées pour la conversion du méthanol en hydrocarbures sont les zéolithes du type ZSM, notamment ZSM-5 et ZSM-11, la zéolithe FU-1, la mordénite modifiée. Mise à part la dernière citée, ces zéolithes sont toutes obtenues à l'issue de leur synthèse avec des rapports SiO$_2$/Al$_2$O$_3$ élevés.

De nombreux brevets concernent la zéolithe ZSM-5 sous forme acide qui est caractérisée par des rapports molaires SiO$_2$/Al$_2$O$_3$ supérieurs à environ 30 et qui, surtout, présente l'avantage de ne pas permettre la formation de composés polyaromatiques précurseurs de coke au sein de sa structure, et donc d'être très stable au cours de la réaction catalytique. Parmi les principaux brevets peuvent ainsi être mentionnés les brevets USA suivants : 3 894 103, 3 894 106, 3 894 107, 3 899 544, 3 928 484, 3 998 899, 4 083 888, 4 083 889. Des informations intéressantes peuvent également être trouvées dans diverses revues scientifiques telles que Journal of Catalysis 47, 249 (1977) Journal of Catalysis 53, 40 (1978) et Journal of Catalysis 61, 155 (1980).

La zéolithe ZSM-5, si elle est très stable, présente l'inconvénient d'une sélectivité médiocre en oléfines légères : elle produit en effet une quantité importante d'hydrocarbures plus lourds allant de C$_5$ à C$_{10}$ essentiellement donc situés dans la coupe essence. Bien que l'essence produite ait un excellent indice d'octane, ce produit est moins intéressant, car valorisable à un moindre prix, que les oléfines légères, notamment éthylène et propylène.

Divers artifices ont été utilisés pour améliorer la sélectivité de la zéolithe ZSM-5, notamment l'addition de phosphore, de silice, de carbone. Certaines de ces améliorations sont décrites dans les brevets précédemment cités.

Plus récemment, un brevet de MOBIL, GB-A-2 093 721, décrit l'utilisation de zéolithes enrichies en silice telles que ZSM-5, ZSM-11, ZSM-12, ZSM-23, ZSM-35, ZSM-38 et ZSM-48. Ces zéolithes sont synthétisées facilement avec des rapports molaires SiO$_2$/Al$_2$O$_3$ élevés, par exemple supérieurs à 30, voire même à 100.

Le brevet US-A-172 857 revendique l'utilisation de zéolithe FU-1 pour convertir le méthanol et/ou le diméthyléther essentiellement en hydrocarbures légers de C$_1$ à C$_4$. Cette zéolithe est comme certaines zéolithes ZSM, à base d'une structure que l'on peut obtenir directement après synthèse avec un rapport SiO$_2$/Al$_2$O$_3$ élevé, égal ou supérieur à 50.

En dehors de quelques zéolithes du type ZSM et de zéolithes du type NU-1 ou FU-1, il est difficile de synthétiser directement des structures zéolithiques avec des rapports $SiO_2/Al_2O_3$ élevés, supérieurs à 12-15 par exemple. C'est encore possible dans le cas de zéolithes n'appartenant pas au groupe des pentasils mais présentant des analogies structurales évidentes avec les zéolithes de ce groupe (elles possèdent par exemple les mêmes unités structurales secondaires 5-1, ainsi que le décrivent les deux articles suivants : W. M. MEIER et D. H. OLSON, Atlas of Zeolite Structure Types 1978, et J. DWYER, A. DYER, Chemistry and Industry, 1984, 2 april, 237-240), telles que la mordénite et la ferriérite. Ainsi, il n'existe pas, à notre connaissance, de mordénite ou de ferriérite présentant directement, après synthèse, un rapport $SiO_2/Al_2O_3$ supérieur à 20 pour la mordénite (D. J. WHITTEMORE Jr., Amer. Mineral., 1972, 57, 1146) et à 70 pour la ferriérite (R. M. BARRER, « Hydrothermale Chemistry of zeolites », Academic Press, London 1982, p. 257). Enfin, les zéolithes appartenant à des groupes autres que les pentasils ou que les groupes apparentés à ce dernier (groupe de la mordénite), telles les zéolites Y, la zéolithe Ω (ou mazzite), l'érionite, l'offrétite, la chabazite, etc... ne peuvent être obtenues qu'exceptionnellement avec des rapports $SiO_2/Al_2O_3$ supérieurs à 12-15 à l'issue de la synthèse hydrothermale. La ZSM-34, revendiquée par la Société MOBIL dans FR-A-2 369 997 semble être une de ces exceptions. Elle appartient à la famille érionite-offrétite, est caractérisée par une morphologie tubulaire différente de la morphologie habituelle de l'érionite, de l'offrétite ou de l'érionite T (cette dernière est le résultat de la cocristallisation des deux zéolithes précédentes en cours de synthèse), et par un rapport $SiO_2/Al_2O_3$ compris entre 10 et 70.

En réalité, comme le mentionnent les brevets US-A-4 079 095 et US-A-4 079 096, la zéolithe ZSM-34 aurait un rapport $SiO_2/Al_2O_3$ inférieur à 50 et semblerait même très difficile à obtenir avec un rapport $SiO_2/Al_2O_3$ supérieur à 20. En effet, les exemples présentés dans les brevets US-A-4 079 095 et US-A-4 079 096, concernent uniquement une zéolithe ZSM-34 dont le rapport $SiO_2/Al_2O_3$ est voisin de 10 (10,2 au maximum). Ceci n'est par surprenant car il est connu dans l'art antérieur qu'il est très difficile d'obtenir un rapport $SiO_2/Al_2O_3$ supérieur à 10-15 avec les techniques actuelles de synthèse pour les structures du type érionite-offrétite. Les durées des tests de conversion du méthanol reportés dans ces brevets atteignent 12 heures au maximum, mais l'activité du catalyseur diminue encore rapidement puisque la production d'hydrocarbures passe de 42,6 % poids après 1,5 heure, à 32 % après 5 heures, à 15 % poids après 7,5 heures et à 8,9 % poids après 11,5 heures, la sélectivité en éthylène étant toutefois excellente puisqu'elle reste toujours proche de ou supérieure à 50 % poids (exprimée par rapport aux hydrocarbures de $C_1$ à $C_5$).

La demande de brevet allemand DE 3 139 355-A 1 mentionne l'utilisation de la zéolithe ZKU, dont les rapports molaires $SiO_2/Al_2O_3$ sont compris entre 8 et 70, pour convertir le méthanol en hydrocarbures légers ($C_1$ à $C_5$ essentiellement) riches en oléfines. Les zéolithes ZKU semblent, d'après les données cristallographiques, appartenir également à la famille érionite-offrétite.

Les traitements modificateurs qui permettent de modifier notablement la composition de la charpente aluminosilicatée, sont une voie de choix pour ajuster les propriétés acides des zéolithes et améliorer leurs performances dans la conversion du méthanol en hydrocarbures. Cependant, les brevets mentionnant l'utilisation de tels traitements sont peu nombreux. Signalons les brevets US-A-3 506 400 et US-A-3 551 353 qui concernent l'application de tels traitements à la mordénite, la faujasite et l'érionite.

Un brevet récent de la société chimique de la Grande Paroisse FR-A-2 519 335, revendique ainsi l'utilisation d'une mordénite fortement enrichie en silice soit par une succession d'attaques acides, soit par une succession de traitements hydrothermiques entre 550 et 680 °C et d'attaques acides alternés, pour la production sélective d'oléfines légères à partir de méthanol.

Dans la présente invention, on a utilisé des zéolithes du type offrétite acide modifiée pour convertir le méthanol, le diméthyléther ou un mélange de ces deux réactifs, en hydrocarbures légers à majorité d'oléfines en $C_2$ et $C_3$. Les offrétites selon l'invention sont définies par la suite.

L'offrétite est une zéolithe, naturelle ou synthétique, appartenant au groupe chabazite. Sa structure a longtemps été considérée comme identique à celle de l'érionite, zéolithe de la même famille, par les similitudes de leurs spectres de diffraction X (HEY M. H. & FEJER E. E., Min. Mag. 33, 66, 1962). Cependant, ces deux structures sont différentes : d'une part, la maille hexagonale de l'offrétite a une dimension suivant l'axe C qui est la moitié de celle de l'érionite (BENETT J. M. & GARD J. A., Nature 214, 1005, 1967), et ainsi, les raies 1 impaires présentes dans les spectres de diffraction X de l'érionite sont absentes dans les spectres de l'offrétite (GARD J. A. & TAIT J. M., Molecular Sieve Zeolites-1, Advan. Chem., Ser. 101, 230, 1971) ; d'autre part, les séquences d'empilement des deux zéolithes sont différentes (WHYTE T. E. Jr., WU E. L., KERR G. T. & VENUTO P. B., J. Catal. 20, 88, 1971). L'offrétite possède ainsi une structure beaucoup plus ouverte que l'érionite. Des défauts d'empilement peuvent survenir dans ces structures, donnant lieu à la formation d'érionite T qui est une zéolithe de structure offrétite avec des défauts d'empilement de type érionite.

La structure de l'offrétite a été précisée par de nombreux auteurs. Cette zéolithe est constituée de deux types de colonnes : des colonnes de cages gmélinites à 14 faces de diamètre 3,6 Å environ ($3,6 \times 10^{-10}$ m) et des colonnes de cages cancrinites à 11 faces alternant avec des prismes hexagonaux. Ces colonnes sont dirigées suivant l'axe C et entraînent la formation de deux types de canaux : des gros canaux dodécagonaux parallèles à l'axe C, de diamètres compris entre 6 et 7 Å (6 à $7 \times 10^{-10}$ m) et des canaux octogonaux perpendiculaires à l'axe C.

L'offrétite est le plus souvent synthétisée en présence d'ions tétraméthylammonium ($(CH_3)_4N^-$ ou

3

TMA) et potassium K+. Les rapports $SiO_2/Al_2O_3$ molaires sont généralement compris entre 4 et 10. D'après la structure décrite plus haut, quatre localisations sont possibles pour ces cations : les prismes hexagonaux, les cages cancrinites, les cages gmélinites et les gros canaux. La répartition de ces cations est la suivante (AIELLO R., BARRER R. M., DAVIES J. A. & KERR I. S., Trans. Faraday Soc. 66, 1610, 1970 ; AIELLO R. & BARRER R. M., Chem. Soc. (A), 1470, 1970) : les ions potassium sont situés dans les prismes hexagonaux, les cages cancrinites et éventuellement les gros canaux ; les ions TMA, plus encombrés, sont situés dans les cages gmélinites et les gros canaux. Il résulte de cette répartition les propriétés d'échanges théoriques suivantes : les ions TMA ou K+ situés dans les gros canaux sont accessibles, donc échangeables, alors que les ions TMA situés dans les cages gmélinites sont « prisonniers » donc non échangeables ; les ions potassium situés dans les cages cancrinites sont à priori accessibles donc échangeables, par contre ceux situés dans les prismes hexagonaux sont inaccessibles donc non échangeables.

L'obtention d'une offrétite stabilisée et enrichie en silice est primordiale dans une réaction telle que la conversion du méthanol, pour améliorer d'une part la sélectivité en oléfines légères et d'autre part la stabilité du catalyseur.

Il n'existe pas actuellement de méthode de synthèse directe d'offrétites riches en silice. On doit donc avoir recours à des modifications de ce solide, c'est-à-dire à la désalumination. La désalumination de l'offrétite semblait impossible jusqu'à présent sans détruire le solide. En effet la structure X de l'offrétite est détruite par un traitement acide.

— Produit offrétite désaluminée.

On a constaté qu'il est possible de préparer une offrétite modifiée, à partir d'une offrétite comportant des cations potassium K+, des ions tétraméthylammonium et éventuellement des cations Na+, cette offrétite, ayant un rapport molaire $SiO_2/Al_2O_3$ compris entre 4 et 10, étant soumise d'abord à au moins un échange cationique précédé éventuellement d'une calcination sous air, en vue d'abaisser la teneur en potassium entre environ 1,5 et 3 % poids, puis étant soumise ensuite à au moins un cycle comportant au moins deux étapes, à savoir (a) au moins un traitement thermique entre 40 et 900 °C et (b) au moins un traitement en phase liquide, ce traitement en phase liquide étant un traitement choisi dans le groupe constitué par un échange cationique (effectué dans au moins une solution d'un sel d'ammonium ionisable, à une température comprise entre 0 et 150 °C) et une attaque acide (effectuée en présence d'un acide minéral ou un acide organique à une température comprise entre 0 et 150 °C), l'étape (b) comportant toujours au moins un échange cationique et au moins une attaque acide, cet échange et cette attaque étant réalisés soit dans un même cycle s'il n'y a qu'un seul cycle soit s'il y a plusieurs cycles dans un même cycle ou dans deux cycles différents ou à la fois dans un même cycle et dans deux cycles différents.

Le produit obtenu a un spectre de diffraction correspondant à celui de l'offrétite, de paramètres cristallins de dimension suivante : a compris entre 1,285 et 1,310 nm et c compris entre 0,748 et 0,755 nm (1 nm = $10^{-9}$ m), une capacité d'adsorption de benzène supérieure à 5 % et de préférence à 7 %, (voir conditions plus loin), une microporosité secondaire, mesurée par la méthode BJH, située entre 3 et 5 nm et correspondant à environ 5 à 50 % du volume poreux total de la zéolithe. (La méthode BJH est explicitée plus loin.)

Tableau 1

| 2 θ | d (nm) | Intensité | 2 θ | d (nm) | Intensité |
|---|---|---|---|---|---|
| 7.65 | 1.153 | 100 | 27.00 | 0.330 | 19 |
| 11.70 | 0.755 | 14 | 28.35 | 0.315 | 26 |
| 13.40 | 0.661 | 29 | 30.55 | 0.292 | 4 |
| 14.05 | 0.629 | 8 | 31.40 | 0.284 | 100 |
| 15.45 | 0.573 | 16 | 33.50 | 0.267 | 13 |
| 19.45 | 0.456 | 27 | | | |
| 20.55 | 0.432 | 41 | | | |
| 23.36 | 0.380 | 31 | | | |
| 23.72 | 0.375 | 78 | | | |
| 24.85 | 0.358 | 41 | | | |

Diagramme de diffraction des rayons X d'une offrétite K-TMA (9,9 % pds K$^+$) de rapport molaire

$$\frac{SiO_2}{Al_2O_3} = 8$$

paramètres a = 1,322 nm
cristallins c = 0,752 nm

— Conversion méthanol.

En partant d'offrétite de rapport SiO$_2$/Al$_2$O$_3$ par exemple voisin de 8 et synthétisée en présence d'ions potassium et de cations tétraméthylammonium, on a préparé une série d'offrétites acides stabilisées, désaluminées et desquelles la plus grande part du potassium a été éliminée par échange ionique. On a constaté avec surprise que la sélectivité en oléfines légères et surtout la stabilité du catalyseur s'améliorent considérablement lorsque le rapport molaire SiO$_2$/Al$_2$O$_3$ atteint des valeurs supérieures à 30 et notamment lorsqu'il atteint des valeurs comprises à l'intérieur de la fourchette 50 à 1 000 de manière préférée de 80 à 300. De manière surprenante, malgré l'appauvrissement important de la charpente aluminosilicatée en aluminium, le nombre des sites acides et leur force restent donc suffisante pour conférer à la zéolithe une très bonne activité lui permettant d'atteindre des conversions du méthanol voisines de 100 % sur plusieurs heures, au-delà de 400 °C, à une vitesse spatiale ppH (poids de méthanol injecté par heure par unité de poids de catalyseur) égale à 2.

Comme écrit ci-dessus, les offrétites dont le rapport SiO$_2$/Al$_2$O$_3$ est inférieur à 30 présentent une activité initiale très élevée pour la conversion du méthanol en hydrocarbures, mais ces catalyseurs se désactivent très rapidement, perdant la quasi totalité de leur activité après quelques minutes ou dizaines de minutes.

Il a donc été observé que la durée de vie de l'offrétite pour la transformation du méthanol en hydrocarbures s'améliore très substantiellement lorsque le rapport SiO$_2$/Al$_2$O$_3$ augmente. En revanche, le nombre de sites acides directement lié au nombre d'atomes d'aluminium dans la charpente aluminosilicatée, diminue évidemment lorsque le rapport SiO$_2$/Al$_2$O$_3$ augmente. Il a été observé que le meilleur compromis entre ces deux variations opposées, correspondant à une activité et une stabilité satisfaisantes, est atteint pour des valeurs du rapport SiO$_2$/Al$_2$O$_3$ comprises entre 80 et 300. De plus, pour ces valeurs, la sélectivité en oléfines légères est bien supérieure à celle obtenue lorsque le rapport SiO$_2$/Al$_2$O$_3$ est inférieur à 30.

— Caractérisation.

L'offrétite riche en silice obtenue dans la présente invention a été caractérisée par les techniques suivantes :

— Diffraction des rayons X.

L'appareillage utilisé comprend : un générateur PHILIPS PW 1130 (35 mA, 35 kV), un goniomètre PHILIPS PW 1050, un tube Cu (Foyer fin), un monochromateur arrière cristal de graphite, un passeur automatique d'échantillon.

A partir des spectres de diffraction des rayons X, on a mesuré pour chaque échantillon, la surface du fond sur une plage (2 θ) de 6 à 32° d'une part, et dans la même zone, la surface des raies en nombre d'impulsions pour un enregistrement pas à pas de 2 secondes sur des pas de 0.02° (2 θ). Le pourcentage de produit cristallisé est exprimé par le rapport surface des raies/surface totale. On compare ensuite les rapports de chaque échantillon traité, par rapport à une référence étalon de la même série que l'échantillon et contenant une quantité inférieure ou égale à 3 % en poids de potassium. Ainsi le taux de cristallinité est exprimé en pourcentage par rapport à une référence prise arbitrairement à 100.

Il est important de bien choisir la référence, car dans certains cas, il peut y avoir une exhaltation ou une diminution d'intensité des raies, en fonction de la teneur en cations des échantillons.

Les paramètres cristallins ont été calculés par la méthode des moindres carrés à partir de la formule (maille hexagonale) :

$$d = \sqrt{\left[\frac{4}{3}a^2(h^2 + k^2 + hk)\right] + \frac{l^2}{c^2}}$$

— Adsorption d'eau.

Chaque échantillon est activé par un préchauffage à 425 °C pendant 16 heures à une pression de $5 \times 10^{-3}$ mmHg dans un appareil conventionnel type Mc Bain. Ensuite, la température de l'échantillon est ajustée à la valeur désirée (25 °C) et l'échantillon est mis en contact avec la vapeur d'eau à la pression désirée (P/P$_0$ = 0,1).

— Adsorption de benzène.

Ce test est effectué sur le même type d'appareil et avec le même type d'activation que pour les mesures d'adsorption d'eau. La température de l'échantillon est fixée à 25 °C et la pression de benzène à environ 70 bars. La durée d'adsorption est fixée à 4 heures.

— Microporosité.

La microporosité est déterminée par la technique BJH (BARRET, JOYNER, HELENDA, J. Am. Chem. Soc. 73, 373 (1951)) basée sur l'exploitation numérique de l'isotherme de désorption d'azote ; le volume poreux total est pris à une pression d'azote telle que $P/P_0 = 0,9$, P étant la pression partielle d'azote de la mesure et $P_0$ la pression de vapeur saturante de l'azote à la température de la mesure.

— Les dosages de potassium, sodium, silicium et aluminium ont été effectués par analyse chimique.

— Obtention du produit.

Le produit de départ est une offrétite synthétique, contenant des ions tétraméthylammonium (TMA) et $K^+$ ($K^+$ par exemple voisin de 10 % pds) ou TMA, $K^+$, $Na^+$ ($0,5 < K/Na + K < 0,8$), et de rapport molaire silice sur alumine compris entre 4 et 10.

L'offrétite de rapport molaire silice sur alumine supérieur à 30 de la présente invention, a été obtenue en effectuant, sur une offrétite partiellement échangée par la technique classique d'échanges cationiques de l'art antérieur, au moins un cycle et de préférence au moins deux cycles successifs traitements thermiques-traitements phase liquide.

Le type de manipulations conformes à l'invention effectuées sur une offrétite contenant des ions TMA et potassium (9,9 % poids) est le suivant :

1) D'abord on prépare un produit selon les techniques de l'art antérieur, contenant un taux de potassium compris entre 1,5 et 3 % en poids, cette technique ne permettant pas de descendre généralement en dessous de 1,5 % poids de potassium ; la méthode est la suivante :

— Elimination des cations TMA par calcination sous air, à des débits compris entre 0,01 et 100 l/h/g, de préférence entre 0,5 et 10 l/h/g et à des températures comprises entre de préférence 450 et 650 °C, et pendant une durée supérieure à une demi-heure.

— Au moins trois échanges cationiques selon la technique classique, à des températures comprises entre 0 et 150 °C, avec un sel d'ammonium ionisable tel que le nitrate, le sulfate, le chlorure, l'acétate d'ammonium, et de molarité comprise entre 0,1 et 3 M, de référence 2 M ; ces échanges sont réalisés avec un rapport volume de solution sur poids de solide sec (V/P) compris entre 3 et 20, de préférence entre 4 et 10.

Le solide obtenu par cette série d'échanges cationiques contient de 1,5 à 3 % en poids de potassium environ.

2) Puis préparation, à partir du produit obtenu par la méthode de l'art antérieur décrite ci-dessus, d'une offrétite enrichie en silice, de rapport molaire silice sur alumine supérieur à 10, par au moins un cycle d'opérations suivantes :

— Calcination sous air, de préférence contenant entre 5 et 100 % de vapeur d'eau, à des débits compris entre 0,01 et 100 l/h/g, de préférence entre 0,5 et 10 l/h/g, à des températures comprises entre 400 et 900 °C, de préférence entre 500 et 800 °C, et pendant une durée supérieure à une demi-heure, de préférence supérieure à une heure.

On peut remplacer la calcination sous air humide par une calcination en atmosphère statique, c'est-à-dire en l'absence de tout débit gazeux, l'humidité étant apportée par le produit lui-même. On peut appeler ce protocole self-steaming ou calcination en atmosphère confinée.

— Au moins un échange cationique dans les conditions décrites plus haut ou au moins une attaque acide à des températures comprises entre 0 et 150 °C, avec un acide minéral tel que l'acide chlorhydrique, l'acide nitrique, l'acide bromhydrique, l'acide sulfurique, l'acide perchlorique, ou organique tel que l'acide acétique ou l'acide benzoïque, de normalité comprise entre 0,1 et 6 N (de préférence entre 0,5 et 2 N), avec un rapport V/P compris entre 3 et 50, de préférence entre 4 et 10, et pendant des durées supérieures à 1/2 heure.

Le procédé de l'invention doit mettre en œuvre ici au moins un échange cationique et au moins une attaque acide.

L'attaque acide est effectuée de façon contrôlée. Le solide est d'abord mis en suspension dans l'eau distillée à une température comprise entre 0 et 150 °C, puis on ajoute goutte à goutte, la quantité d'acide théorique nécessaire pour extraire une quantité voulue d'aluminium. De préférence, on se place en défaut d'ions $H^+$ par rapport à la quantité d'aluminium total contenue dans le solide. Une fois l'ajout d'acide terminé, on laisse sous agitation pendant une durée supérieure à une demi-heure. On pourra ainsi obtenir selon l'invention une offrétite modifiée de rapport molaire $SiO_2/Al_2O_3$ supérieur à 30 à partir d'une offrétite de rapport molaire $SiO_2/Al_2O_3$ compris entre 4 et 10 contenant de 1,5 à 3 % de potassium par le cycle d'opérations suivantes, conduit après l'échange cationique préalable :

— calcination sous air humide ou self-steaming.
— traitement par un acide
et/ou encore par le cycle d'opérations suivantes :
— calcination sous air humide ou self steaming.
— échange cationique par des ions ammonium.
— calcination sous air humide ou self-steaming.
— traitement par un acide.

Il est donc possible que le premier traitement en phase liquide soit une attaque acide. Cependant celle-ci devra être menée avec beaucoup de précautions, c'est-à-dire à un pH pas trop faible et une température pas trop élevée pour ne pas affecter la cristallinité du solide et on préférera en général, surtout pour des offrétites de rapport molaire $SiO_2/Al_2O_3$ de départ relativement faible, que le premier traitement en phase liquide soit un échange cationique. Il est également possible d'effectuer un traitement en phase liquide qui soit un traitement mixte échange cationique/attaque acide en mélangeant un acide avec un sel d'ammonium.

L'optimisation de la méthode, le choix de ou des premiers traitements en phase liquide, le nombre de cycles calcination-traitement phase liquide à effectuer, dépendent du rapport molaire $SiO_2/Al_2O_3$ initial, lequel joue sur la stabilité du produit, et du rapport molaire $SiO_2/Al_2O_3$ final. Ainsi le nombre d'opérations nécessaires pour parvenir à un rapport $SiO_2/Al_2O_3$ donné sera d'autant plus grand que le rapport initial sera faible, et que le rapport final sera élevé.

Ainsi l'invention concerne des offrétites acides enrichies en silice qui sont caractérisées par les propriétés suivantes :

— rapport molaire $SiO_2/Al_2O_3$ supérieur à 30
— paramètres cristallins $\vec{a}$ et $\vec{c}$ compris respectivement
pour $\vec{a}$ entre 1,285 et 1,310 nm
pour $\vec{c}$ entre 0,748 et 0,755 nm
— teneur en potassium inférieure à 1 % en poids
— capacité d'adsorption de benzène à 25 °C, à une pression partielle de 70 torrs (133, 32 × 70 Pa) supérieure à 5 % en poids
— existence d'une microporosité secondaire, mesurée par la méthode BJH, située entre 3 et 5 nm et correspondant à environ 5 à 50 % du volume poreux total de la zéolithe, lequel est déterminé par adsorption d'azote pour un rapport $P/P_0$ de 0,9.

Ces produits ainsi caractérisés, sont utilisables pour convertir le méthanol ou le diméthyléther en un mélange d'hydrocarbures.

On a découvert que les catalyseurs les plus adaptés à la production d'un mélange riche en oléfines légères $C_2$-$C_4$, et les catalyseurs les plus stables étaient constitués de zéolithes fortement enrichies en silice. Plus précisément ces offrétites ont les caractéristiques suivantes :

— rapport molaire $SiO_2/Al_2O_3$ supérieur de préférence à 50 (notamment entre 50 et 1 000 et plus particulièrement entre 80 et 300).
— paramètres cristallins $\vec{a}$ et $\vec{c}$ compris respectivement
pour $\vec{a}$ entre 1,285 et 1,305 nm et de préférence entre 1,290 et 1,300 nm
pour $\vec{c}$ entre 0,748 et 0,755 nm.
— teneur en potassium inférieure à 1 % en poids et de préférence à 0,5 % en poids.
— capacité d'adsorption de benzène, à 25 °C et à une pression partielle de 70 torrs, supérieure à 5 et de préférence à 6 % en poids.
— capacité d'adsorption d'eau, à 25 °C pour un rapport $P/P_0$ de 0,1, inférieure à 10 % et de préférence inférieure à 8 % en poids.
— existence d'une microporosité secondaire, mesurée par la méthode BJH, située entre 3 et 5 nm et correspondant à environ 5 à 50 % du volume poreux total de la zéolithe.

Les zéolithes acides du type offrétite modifiée ainsi obtenues, peuvent être utilisées pures sous forme de poudre dans la réaction de transformation du méthanol. Elles sont plus avantageusement utilisées sous forme de grains cylindriques, sphériques, ou de forme quelconque de dimension comprise entre environ 0,1 et 10 mm. Elles peuvent là aussi être utilisées pures mais pour des commodités de mise en forme, il peut être avantageux d'ajouter à la zéolithe un liant inerte qui aura pour but de conférer au solide une bonne résistance mécanique. Parmi les liants utilisables, on peut citer par exemple : l'alumine, la silice, les silice-alumines, les argiles ou divers ciments tels que des aluminates d'alcalino-terreux. La teneur en zéolithe dans le catalyseur final sera avantageusement comprise entre 5 et 100 % en poids et de préférence entre 40 et 100 % en poids.

Les catalyseurs obtenus sont utilisables pour transformer le méthanol ou le diméthyléther ou un mélange de ces deux réactifs, en hydrocarbures comprenant une forte proportion d'hydrocarbures légers insaturés et notamment en éthylène, propylène et butènes, dans les conditions opératoires suivantes :

— Pression totale comprise entre 0,1 MPa (0,1 mégapascal ou pression atmosphérique) et 3 MPa (3 mégapascals ou 30 bars) et de préférence entre 0,1 et 2 MPa.

— Température comprise entre 300 et 600 °C et de préférence entre 350 et 550 °C.

— Vitesse spatiale d'alimentation exprimée en grammes de méthanol et/ou diméthyléther par heure et par gramme de catalyseur, comprise entre 0,1 et 10 et de préférence entre 0,5 et 5.

Le méthanol ou le diméthyléther, ou le mélange des deux peut être injecté sur la zéolithe soit pur, soit dilué dans un gaz porteur qui peut être indifféremment par exemple l'hydrogène, l'azote, la vapeur d'eau, le monoxyde de carbone ou le dioxyde de carbone ou encore un mélange de deux ou plusieurs de ces divers gaz. La concentration en méthanol ou diméthyléther ou du mélange de ces deux réactifs dans le gaz porteur est avantageusement supérieure à 10 % en volume.

Après séparation des hydrocarbures formés, le gaz porteur est recyclé ; le méthanol et le diméthyléther non transformés sont également recyclables.

Les exemples suivants illustrent la présente invention. Les exemples 1 et 2 décrivent la préparation de catalyseurs non conformes à l'invention. Les exemples 3 à 8 décrivent la préparation de catalyseurs conformes à l'invention. Les exemples 9 à 11 présentent les performances de quelques catalyseurs des exemples précédents dans la réaction de conversion du méthanol, dans des conditions opératoires qui sont décrites plus loin.

## Exemple 1 (comparatif)

Préparation d'une offrétite non conforme à l'invention.

200 g d'une offrétite synthétique de rapport molaire $SiO_2/Al_2O_3$ égal à 8 contenant 9,9 % en poids de potassium et 2,8 % en poids d'ions tétraméthylammonium ont été calcinés sous air sec, à un débit de 3 l/h/g, pendant 2 heures à 550 °C, pour éliminer les cations $TMA^+$.

Le produit obtenu (référencé 1 A) a ensuite été échangé trois fois par une solution 2 M de nitrate d'ammonium, à 100 °C pendant 4 heures, sous agitation, avec un rapport volume de solution sur poids de solide sec (V/P) égal à 5.

Les produits obtenus sont référencés 1 B, 1 C et 1 D. Les taux de potassium obtenus sont les suivants :

| | produit de départ | 1er échange (1 B) | 2ème échange (1 C) | 3ème échange (1 D) |
|---|---|---|---|---|
| $K^+$ (% poids) | 9.9 | 4.2 | 3.0 | 2.3 |

Le produit 1 D obtenu a les caractéristiques suivantes :

| | Diffraction des rayons X | | | Adsorption |
|---|---|---|---|---|
| | S raies($10^3$) | S fond ($10^3$) | S raies/((%) S totale | $H_2O$ |
| 1 D | 277 | 203 | 58 | 22.4 |

Le rapport molaire $SiO_2/Al_2O_3$ est de 8.

Le diagramme de diffraction du produit 1 D ($NH_4$-offrétite) est présenté dans le tableau 2.

(Voir Tableau 2 page 9)

## EP 0 190 949 B1

Tableau 2

Diagramme de diffraction X de $NH_4$-offrétite

| 2 θ | d (nm) | Intensité | 2 θ | d (nm) | Intensité |
|---|---|---|---|---|---|
| 7.70 | 1.145 | 66 | 28.10 | 0.317 | 10 |
| 11.75 | 0.752 | 7 | 28.40 | 0.314 | 24 |
| 13.40 | 0.661 | 37 | 30.55 | 0.292 | 5 |
| 14.10 | 0.628 | 7 | 31.25 | 0.286 | 62 |
| 15.50 | 0.572 | 18 | 31.50 | 0.284 | 59 |
| 17.85 | 0.496 | 2 | 33.50 | 0.267 | 24 |
| 19.50 | 0.455 | 26 | | | |
| 20.50 | 0.433 | 54 | | | |
| 23.31 | 0.382 | 31 | | | |
| 23.70 | 0.375 | 100 | | | |
| 24.90 | 0.357 | 66 | | | |
| 26.20 | 0.340 | < 1 | | | |
| 27.00 | 0.330 | 23 | | | |
| 27.30 | 0.327 | 7 | | | |

### Exemple 2 (comparatif)

Préparation d'un catalyseur non conforme à l'invention.

25 g d'offrétite issue de l'exemple précédent, référencée 1 D ont été soumis à deux attaques acides successives par HCl 0,4 N à 100 °C pendant 4 heures, avec un rapport V/P de 20.

Les produits obtenus sont référencés 2 A (1$^{ère}$ attaque acide) et 2 B (2$^{eme}$ attaque acide) et les résultats obtenus sont présentés dans le tableau ci-dessous :

| | | 1 D | 2 A | 2 B |
|---|---|---|---|---|
| $K^+$ (% poids) | | 2.8 | 1.6 | 0.4 |
| $SiO_2/Al_2O_3$ (mol.) | | 8 | 12.5 | 52.4 |
| Diffraction X | S raies $(10^3)$ | 277 | 266 | 76 |
| | S fond $(10^3)$ | 203 | 375 | 399 |
| | S raies/ S totale (%) | 58 | 41.5 | 16 |
| | Cristallinité (%) | 100 | 72 | 28 |

Cet exemple montre l'instabilité de l'offrétite en milieu acide, la structure se dégradant considérablement à la deuxième attaque acide.

### Exemple 3 (non conforme à l'invention)

Préparation d'une offrétite de rapport molaire $SiO_2/Al_2O_3$ égal à 25.

Sur l'offrétite obtenue à l'exemple 1 contenant 2,8 % en poids de potassium (référencée 1 D), on a effectué les opérations suivantes :

9

Premier cycle :

— Self-steaming à 550 °C pendant 2 heures (produit 3 A).
— 2 échanges cationiques successifs par $NH_4NO_3$ 2 M dans les conditions décrites dans l'exemple 1 (produits 3 B et 3 C).

Deuxième cycle :

— Self-steaming à 650 °C pendant 2 heures (produit 3 D).
— Echange cationique par $NH_4NO_3$ 2 M dans les conditions décrites dans l'exemple 1 (produit 3 E).
— 2 attaques acides successives par HCl 0,23 N puis HCl 0,36 N à 100 °C pendant 4 heures, avec un rapport V/P égal à 10 (produits 3 F et 3 G).

|  | | 1D | 3A | 3B | 3D | 3E | 3F | 3G |
|---|---|---|---|---|---|---|---|---|
| $SiO_2/Al_2O_3$ (mol) | | 8 | | | | | | 25 |
| Diffraction X | Sraies($10^3$) | 277 | 303 | 323 | 298 | 274 | 290.5 | 285 |
| | Sfond ($10^3$) | 203 | 193 | 209 | 240 | 256.5 | 269 | 256 |
| | Sraies/ S totale (%) | 58 | 61 | 61 | 55 | 52 | 52 | 53 |
| | Cristalli- nité (%) | 100 | 105 | 105 | 95 | 90 | 90 | 91 |
| | paramètres (Å) a | 13.22 | 13.10 | 13.16 | 13.02 | 13.02 | 13.01 | 13.03 |
| | c | 7.52 | 7.50 | 7.51 | 7.51 | 7.51 | 7.51 | 7.52 |

(1 Å = $10^{-10}$ m).

A l'issue de ces divers traitements, la cristallinité du produit reste toujours excellente (son diagramme de diffraction est présenté dans le tableau 3), sa teneur en potassium est de 0,7 % en poids, son rapport $SiO_2/Al_2O_3$ est égal à 25, et sa capacité d'adsorption d'eau est de 15 % (P/P₀ = 0,1). Le solide 3 G est appelé catalyseur 1.

Tableau 3

Diagramme de diffraction X du catalyseur 1

| 2 θ | d (nm) | Intensité | 2 θ | d (nm) | Intensité |
|---|---|---|---|---|---|
| 7.85 | 1.13 | 80 | 24.0 | 0.371 | 44 |
| 11.80 | 0.751 | 7 | 24.95 | 0.356 | 47 |
| 13.60 | 0.650 | 100 | 27.40 | 0.325 | 27 |
| 14.15 | 0.625 | 43 | 28.50 | 0.313 | 20 |
| 15.70 | 0.564 | 33 | 30.95 | 0.289 | 14 |
| 18.00 | 0.492 | 2 | 31.70 | 0.282 | 71 |
| 19.70 | 0.451 | 16 | 33.90 | 0.264 | 22 |
| 20.85 | 0.426 | 57 | | | |
| 23.70 | 0.375 | 29 | | | |

## Exemple 4

Préparation d'un catalyseur conforme à l'invention.

Sur l'offrétite appelée catalyseur 1 obtenue à l'exemple 3 de rapport molaire silice sur alumine de 25 (référencée 3 G), on a effectué les opérations suivantes :

— Self-steaming à 750 °C pendant 2 heures (produit 4 A).
— Echange cationique par $NH_4NO_3$ 2 M dans les conditions décrites dans l'exemple 1 (produit 4 B).
— 1 attaque acide par HCl 0,61 N (V/P = 15) à 100 °C pendant 4 heures (produit 4 C).

| | | 1 D | 3 G | 4 A | 4 B | 4 C |
|---|---|---|---|---|---|---|
| $SiO_2/Al_2O_3$ (mol.) | | 8 | 25 | | | 67.2 |
| Diffraction X | S raies ($10^3$) | 277 | 285 | 306 | 316 | 330 |
| | S fond ($10^3$) | 203 | 256 | 248 | 255 | 259 |
| | S raies/ S totale (%) | 58 | 53 | 55 | 55 | 56 |
| | Cristallinité (%) | 100 | 91 | 95 | 95 | 97 |
| | Paramètres a Å | 13.22 | 13.03 | 12.93 | 12.93 | 12.93 |
| | c | 7.52 | 7.52 | 7.51 | 7.53 | 7.53 |

(1 Å = $10^{-10}$ m).

Le produit solide 4 C ainsi obtenu de rapport molaire $SiO_2/Al_2O_3$ de 67 présente une excellente cristallinité (son diagramme de diffraction est présenté dans le tableau 4). Sa teneur en potassium est de 0,3 % poids, sa capacité d'adsorption d'eau est de 5 % à un rapport $P/P_0$ de 0,1, et il présente une microporosité secondaire à des diamètres compris entre 20 et 60 Å, (20 et 60 × $10^{-10}$ m) correspondant à 0,10 $cm^3$/g pour un volume poreux total mesuré à un rapport $P/P_0$ de 0,9 de 0,38 $cm^3$/g. Ce produit 4 C est appelé catalyseur 2.

### Tableau 4

### Diagramme de diffraction X du catalyseur 2

| 2 θ | d nm | intensité | 2 θ | d nm | Intensité |
|---|---|---|---|---|---|
| 7.90 | 1.12 | 100 | 31.85 | 0.281 | 40 |
| 11.80 | 0.751 | 5 | 34.10 | 0.2625 | 13 |
| 13.70 | 0.645 | 90 | | | |
| 14.20 | 0.624 | 39 | | | |
| 15.85 | 0.559 | 20 | | | |
| 18.10 | 0.490 | 1 | | | |
| 19.75 | 0.449 | 15 | | | |
| 21.00 | 0.423 | 36 | | | |
| 23.90 | 0.372 | 18 | | | |
| 24.15 | 0.368 | 22 | | | |
| 24.95 | 0.356 | 25 | | | |
| 27.55 | 0.323 | 15 | | | |
| 28.55 | 0.312 | 9 | | | |
| 31.10 | 0.287 | 7 | | | |

## Exemple 5

Préparation d'un catalyseur conforme à l'invention.

100 grammes du catalyseur 2 précédent, sont soumis à une attaque supplémentaire de 4 heures dans 1 litre d'une solution 3,33 M d'acide ClH à 100 °C.

Le produit obtenu présente une excellente cristallinité, comparable à celle du catalyseur 2. Les paramètres cristallins de la maille élémentaire sont a = 1,292 nm c = 0,753 nm. Sa teneur en potassium est de 0,25 % en poids. Son rapport $SiO_2/Al_2O_3$ est égal à 190 environ. Ce produit est appelé catalyseur 3.

## Exemple 6

Préparation d'un catalyseur conforme à l'invention.

50 grammes du catalyseur 3 précédent sont soumis à un quatrième « self-steaming » de 3 heures à 750 °C, puis à deux attaques acides successives dans 250 cm³ d'une solution 1 M d'acide chlorhydrique, à 90 °C pendant 4 heures.

Le produit obtenu est encore très bien cristallisé : sa cristallinité est quasiment identique à celle du catalyseur 3. Les paramètres cristallins de la maille élémentaire de ce solide sont a = 1,289 nm c = 0,753 nm. Sa teneur en potassium est égale à 0,08 % en poids. Son rapport $SiO_2/Al_2O_3$ est égal à 250 environ. Ce produit est appelé catalyseur 4.

## Exemple 7

Préparation d'un catalyseur conforme à l'invention.

Sur l'offrétite obtenue à l'exemple 1 contenant 2,8 % en poids de potassium (référencée 1 D), on a effectué 2 cycles successifs des opérations suivantes :

— Self-steaming à 650 °C (1er cycle) et 750 °C (2e cycle) pendant 2 heures (produits 7 A et 7 D).
— Echange cationique par $NH_4NO_3$ 2 M dans les conditions décrites dans l'exemple 1 (produits 7 B et 7 E).
— Attaque acide par HCl 0,36 N (1er cycle) et HCl 1 N (2e cycle) à 100 °C pendant 4 heures, avec un rapport V/P égal à 10 (produits 7 C et 7 F).

| | | 1D | 7A | 7B | 7C | 7D | 7E | 7F |
|---|---|---|---|---|---|---|---|---|
| K (% poids) | | 2.8 | | 1.3 | | | 0.67 | |
| $SiO_2/Al_2O_3$ (mol.) | | 8 | | | 12 | | | 68 |
| S raies ($10^3$) | | 277 | 279.5 | 273 | 286 | 265.5 | 253.5 | 268.5 |
| Diffraction X | S fond ($10^3$) | 203 | 216 | 216.5 | 229 | 214.5 | 233.5 | 227.5 |
| | S raies/ (%) S totale | 58 | 56 | 56 | 55.5 | 55 | 52 | 54 |
| | Cristallinité (%) | 100 | 97 | 97 | 96 | 95 | 90 | 93 |
| | Paramètres (Å) a | 13.22 | 13.04 | 13.02 | 13.06 | 12.98 | 12.97 | 12.96 |
| | Paramètres (Å) c | 7.52 | 7.50 | 7.49 | 7.52 | 7.52 | 7.53 | 7.53 |

Le produit obtenu (référencé 7 F) de rapport molaire $SiO_2/Al_2O_3$ égal à 68, est très bien cristallisé et a une capacité d'adsorption du benzène de 10,9 %.

## Exemple 8

Sur l'offrétite obtenue à l'exemple 7 de rapport molaire silice sur alumine de 68 (référencée 7 F), on a effectué un cycle des opérations suivantes :

— Self-steaming à 800 °C pendant 2 heures (produit 8 A).
— Echange cationique par NH₄NO₃ 2 M dans les conditions décrites dans l'exemple 1 (produit 8 B).
— Attaque acide par HCl 3,17 N à 100 °C pendant 4 heures, avec un rapport V/P égal à 10 (produit 8 C).

| | | 1D | 7F | 8A | 8B | 8C |
|---|---|---|---|---|---|---|
| K (% poids) | | 2.8 | | | 0.01 | |
| SiO₂/Al₂O₃ (mol.) | | 8 | 68 | | | 101 |
| Diffraction X | S raies (10³) | 277 | 268,5 | 207 | 201 | 211.5 |
| | S fond (10³) | 203 | 277.5 | 178.5 | 167.5 | 170 |
| | S raies / S totale (%) | 58 | 54 | 54 | 54.5 | 55.5 |
| | Cristallinité (%) | 100 | 93 | 93 | 94 | 96 |
| | Paramètres (Å) a | 13.22 | 12.96 | 12.97 | 12.95 | 12.97 |
| | c | 7.52 | 7.53 | 7.55 | 7.54 | 7.55 |

Le produit obtenu (référencé 8 C) de rapport molaire $SiO_2/Al_2O_3$ de 101, est très bien cristallisé, a une capacité d'adsorption d'eau de 3,6 % ($P/P_0 = 0,1$) une capacité d'adsorption de benzène de 7,9 % et une capacité de reprise de potassium de 0,33 %.

### Exemple 9 (comparatif)

10 g de l'offrétite non conforme à l'invention obtenue à l'exemple 1, référencée 1 G, de rapport molaire $SiO_2/Al_2O_3$ égal à 8, contenant 2,8 % en poids de potassium sont soumis dans un microréacteur à un test de conversion du méthanol en hydrocarbures dans les conditions suivantes :

Pression atmosphérique
Température = 460 °C
Vitesse spatiale d'alimentation (ppH) = 2 (exprimée en gramme de méthanol passant en 1 heure sur 1 gramme de catalyseur)
Gaz porteur = azote
Rapport volumique gazeux azote/méthanol = 3, soit une pression partielle de méthanol de 0,25 bar.

L'évolution de l'activité dans le temps de ce catalyseur est présentée dans le tableau 5. Dans ce tableau, les significations des titres des colonnes sont les suivantes.

DME = diméthyléther
$C_1$ : méthane, $C_2$ : éthane, $C_2^=$ : éthylène,
$C_3$ : propane, $C_3^=$ : propylène, $C_4$ : butanes,
$C_4^=$ : butènes, $C_5^-$ : ensemble des hydrocarbures comprenant 5 ou plus atomes de carbone.

Ces divers composés sont exprimés en pour cent.
La sélectivité en oléfines est le pourcentage d'oléfines dans les hydrocarbures formés (donc compte non tenu du méthanol et du DME présents dans les effluents).
La sélectivité en propylène est le pour cent de propylène dans les hydrocarbures formés.

13

Tableau 5

| Durée de fonctionnement | CH$_3$OH non transformé | DME | C$_1$ | C$_2$ | C$_2$= | C$_3$ | C$_3$= | C$_4$ | C$_4$= | C$_5^+$ | Sélectivité | |
|---|---|---|---|---|---|---|---|---|---|---|---|---|
| | | | | | | | | | | | C$_3$= | oléfines |
| 0.08 | 0 | 0 | 5.0 | 0.8 | 27.9 | 17.0 | 25.8 | 7.9 | 11.1 | 4.5 | 25.8 | 64.8 |
| 1 | 43.1 | 7.8 | 3.8 | 0.7 | 13.8 | 9.2 | 10.7 | 3.9 | 5.2 | 1.8 | 21.8 | 60.5 |
| 2 | 61.6 | 16.4 | 3.2 | 0.6 | 7.4 | 4.3 | 3.4 | 1.4 | 1.5 | 0.2 | 15.5 | 55.9 |
| 4 | 74.1 | 24.3 | 1.1 | 0.1 | 0.4 | 0 | 0 | 0 | 0 | 0 | 0 | 25 |

Cet exemple montre l'instabilité catalytique d'une offrétite non conforme à l'invention.

Exemple 10

Utilisation des catalyseurs 1 à 4 pour convertir le méthanol en hydrocarbures légers insaturés.

10 g de chacun des catalyseurs 1 à 3 sont successivement soumis dans un microréacteur à un test de conversion du méthanol en hydrocarbures dans les mêmes conditions que l'exemple 9.

En ce qui concerne le catalyseur 4, la température du test a été maintenue à 500 °C.

L'évolution de l'activité dans le temps des catalyseurs 1 à 4 est présentée dans les tableaux 6 à 9. Dans ces tableaux, les significations des titres des colonnes sont les mêmes que celles de l'exemple 9, ainsi que les définitions des sélectivités en propylène et en oléfines.

Tableau 6

T = 460 °C   CATALYSEUR 1

| Durée de fonctionnement(h) | CH$_3$OH non transformé | DME | C$_1$ | C$_2$ | C$_2$= | C$_3$ | C$_3$= | C$_4$ | C$_4$= | C$_5^+$ | Sélectivité | |
|---|---|---|---|---|---|---|---|---|---|---|---|---|
| | | | | | | | | | | | C$_3$= | oléfines |
| 1 | 9.2 | 9.9 | 9.3 | 2.8 | 22.6 | 7.8 | 15.8 | 4.8 | 11.9 | 5.9 | 19.5 | 62.2 |
| 2 | 19.7 | 14.0 | 7.5 | 2.1 | 17.5 | 6.1 | 12.1 | 5.6 | 8.5 | 6.9 | 18.2 | 57.5 |
| 4 | 50.8 | 23.5 | 1.8 | 0.8 | 4.7 | 3.5 | 6.2 | 1.9 | 3.7 | 3.1 | 24.1 | 56.8 |
| 8 | 70.1 | 28.7 | 1.2 | 0 | 0 | 0 | 0 | 0 | 0 | 0 | 0 | 0 |
| 12 | 71.8 | 27.1 | 1.1 | 0 | 0 | 0 | 0 | 0 | 0 | 0 | 0 | 0 |
| 20 | 72.1 | 27.0 | 0.9 | 0 | 0 | 0 | 0 | 0 | 0 | 0 | 0 | 0 |

Tableau 7

T = 460 °C   CATALYSEUR 2

| Durée de fonctionnement(h) | CH$_3$OH non transformé | DME | C$_1$ | C$_2$ | C$_2$= | C$_3$ | C$_3$= | C$_4$ | C$_4$= | C$_5^+$ | Sélectivité | |
|---|---|---|---|---|---|---|---|---|---|---|---|---|
| | | | | | | | | | | | C$_3$= | oléfine |
| 1 | 0 | 0 | 6.1 | 0.7 | 12.3 | 5.4 | 35.3 | 7.6 | 17.4 | 15.2 | 35.3 | 65 |
| 2 | 0.2 | 0 | 6.0 | 0.6 | 7.7 | 3.2 | 38.5 | 6.4 | 19.7 | 17.7 | 38.6 | 66 |
| 4 | 1 | 0 | 5.5 | 0.6 | 3.6 | 1.2 | 38 | 4 | 24 | 22.1 | 38.4 | 66.3 |
| 8 | 3.9 | 9.9 | 3.9 | 0.4 | 1.9 | 0.4 | 30.5 | 3.1 | 23.4 | 22.6 | 35.4 | 64.7 |
| 12 | 12 | 23.4 | 3 | 0.4 | 1.4 | 0.6 | 19.2 | 2 | 18.1 | 19.9 | 29.7 | 59.9 |
| 20 | 23.3 | 42.8 | 2.7 | 0.2 | 1.1 | 0.5 | 5.9 | 2 | 11.1 | 10.4 | 17.4 | 53.4 |

Tableau 8

T = 460 °C   CATALYSEUR 3

| durée de fonction-nement(h) | CH$_3$OH non transformé | DME | C$_1$ | C$_2$ | C$_2^=$ | C$_3$ | C$_3^=$ | C$_4$ | C$_4^=$ | C$_5^+$ | Sélectivité | |
|---|---|---|---|---|---|---|---|---|---|---|---|---|
| | | | | | | | | | | | C$_3^=$ | olé-fine |
| 1 | 0 | 0 | 3.9 | 0.4 | 8 | 2 | 43.2 | 4.9 | 22.3 | 15.3 | 43.2 | 73.5 |
| 2 | 0.1 | 0 | 2.9 | 0.2 | 4.2 | 1 | 45.5 | 5.2 | 23.9 | 17 | 45.5 | 73.7 |
| 4 | 0.5 | 0 | 2.3 | 0.2 | 3.2 | 0.7 | 44.8 | 4.6 | 24.9 | 18.8 | 45.0 | 73.3 |
| 8 | 2 | 0 | 2.2 | 0.2 | 2.2 | 0.4 | 43.5 | 3.3 | 25.8 | 20.4 | 44.4 | 73.0 |
| 12 | 4.3 | 8.1 | 1.9 | 0.2 | 1.5 | 0.2 | 34.6 | 2.5 | 25.5 | 21.2 | 39.5 | 70.3 |
| 20 | 11.8 | 31.3 | 1.5 | 0.2 | 1 | 0.2 | 17.2 | 1 | 18.5 | 17.3 | 30.2 | 64.5 |

Tableau 9

T = 500 °C   CATALYSEUR 4

| Durée de fonction-nement(h) | CH$_3$OH non transformé | DME | C$_1$ | C$_2$ | C$_2^=$ | C$_3$ | C$_3^=$ | C$_4$ | C$_4^=$ | C$_5^+$ | Sélectivité | |
|---|---|---|---|---|---|---|---|---|---|---|---|---|
| | | | | | | | | | | | C$_3^=$ | olé-fine |
| 1 | 3.5 | 5.2 | 1.7 | 0.1 | 4.5 | 1.5 | 36.7 | 3.5 | 24.8 | 18.5 | 40.2 | 72.3 |
| 2 | 2.9 | 5 | 1.5 | 0.1 | 3.5 | 0.5 | 41.6 | 2.8 | 25.7 | 16.4 | 45.2 | 76.9 |
| 4 | 4.5 | 8.5 | 1.2 | 0.1 | 2.3 | 0.2 | 41.2 | 2.4 | 23.9 | 15.7 | 47.4 | 77.5 |
| 8 | 5 | 9.5 | 1.2 | 0.2 | 1.7 | 0.3 | 41.2 | 1.9 | 21.8 | 17.2 | 48.2 | 75.7 |
| 12 | 7 | 13.1 | 1.0 | 0.1 | 1.1 | 0.2 | 36.6 | 1.7 | 22.4 | 16.8 | 45.8 | 76.0 |
| 20 | 9 | 19.5 | 0.8 | 0.1 | 0.8 | 0.2 | 32.2 | 1.0 | 20.5 | 15.9 | 45.0 | 74.8 |

Ces résultats montrent que les catalyseurs constitués d'une offrétite acide dont les rapports molaires SiO$_2$/Al$_2$O$_3$ sont supérieurs à 50 ont des performances catalytiques nettement améliorées (tableaux 7 à 9) par rapport à celles que donne une offrétite de rapport SiO$_2$/Al$_2$O$_3$ égal à 8 et même 25. Le catalyseur 4 est le plus sélectif et le plus stable, mais son activité est nettement inférieure à celle des catalyseurs 2 et 3, ce qui a nécessité le choix d'une température de test supérieure à celle des autres tests (500 °C au lieu de 460 °C).

Exemple 11

Essai de régénération du catalyseur 2 conforme à l'invention.

Après un test de 20 heures dans les conditions décrites précédemment (voir exemple 10), le catalyseur 2 a été régénéré à 520 °C sous air dilué par de l'azote pendant 5 heures. Il a ensuite été de nouveau soumis à un test de conversion du méthanol dans les mêmes conditions que précédemment. Les résultats obtenus au cours du temps sont présentés dans le tableau ci-après.

Tableau 10

T = 460 °C   CATALYSEUR 2 régénéré

| Durée de fonction-nement(h) | CH$_3$OH non transformé | DME | C$_1$ | C$_2$ | C$_2^=$ | C$_3$ | C$_3^=$ | C$_4$ | C$_4^=$ | C$_5^+$ | Sélectivité C$_3^=$ | Sélectivité oléfine |
|---|---|---|---|---|---|---|---|---|---|---|---|---|
| 1 | 0 | 0 | 5.1 | 0.4 | 6.1 | 2.7 | 37.6 | 10.8 | 18.6 | 18.7 | 37.6 | 62.3 |
| 2 | 0 | 0 | 4.1 | 0.4 | 5.1 | 2.5 | 39 | 9.9 | 19.3 | 19.7 | 39 | 63.4 |
| 4 | 0.2 | 0 | 3.9 | 0.4 | 3.9 | 1.7 | 40.6 | 8.2 | 20.2 | 20.9 | 40.7 | 64.8 |
| 8 | 3.1 | 0 | 3.5 | 0.4 | 2.6 | 1.2 | 40.2 | 6.1 | 22 | 20.9 | 41.5 | 66.9 |
| 12 | 6 | 9.5 | 3 | 0.2 | 1.9 | 0.6 | 31 | 4.5 | 22.1 | 21.2 | 36.7 | 65.1 |
| 20 | 13.7 | 26 | 1.9 | 0.1 | 0.9 | 0.4 | 19.4 | 2.7 | 17.5 | 17.4 | 32.2 | 62.7 |

Au vu des résultats du tableau, il apparaît que le catalyseur 2 conforme à l'invention présente après régénération des performances légèrement supérieures à celle observée sur le même catalyseur neuf. Ce catalyseur est donc parfaitement régénérable.

**Revendications**

1. Offrétite dont les caractéristiques sont :

— rapport molaire SiO$_2$/Al$_2$O$_3$ supérieur à 30
— paramètres cristallins $\vec{a}$ et $\vec{c}$ compris respectivement entre 1,285 et 1,310 nm pour $\vec{a}$ et entre 0,748 et 0,755 nm pour $\vec{c}$.
— teneur en potassium inférieure à 1 % en poids
— capacité d'adsorption de benzène à 25 °C, à une pression partielle de 70 torrs (70 × 133,32 Pa), supérieure à 5 % en poids
— microporosité secondaire, située entre 3 et 5 nm et correspondant à environ 5 à 50 % du volume poreux total de la zéolithe de structure offrétite.

2. Offrétite selon la revendication 1 dont les paramètres cristallins $\vec{a}$ et $\vec{c}$ sont compris respectivement entre 1,285 et 1,305 nm pour $\vec{a}$ et entre 0,748 et 0,755 nm pour $\vec{c}$.

3. Offrétite selon la revendication 2, la dite offrétite ayant les caractéristiques suivantes :

— rapport molaire SiO$_2$/Al$_2$O$_3$ supérieur à 50
— paramètres cristallins $\vec{a}$ et $\vec{c}$ compris entre 1,290 et 1,300 nm pour $\vec{a}$ et entre 0,748 et 0,755 nm pour $\vec{c}$
— teneur en potassium inférieure à 0,5 % en poids
— capacité d'adsorption de benzène, à 25 °C et à une pression de 70 torrs (133,32 × 70 Pa), supérieure à 6 % en poids
— existence d'une microporosité secondaire, située entre 3 et 5 nm et correspondant à environ 5 à 50 % du volume poreux total de la zéolithe de structure offrétite.

4. Procédé d'obtention d'une offrétite modifiée définie selon la revendication 1, la dite offrétite modifiée étant préparée à partir d'une offrétite comportant des cations potassium K$^-$, des ions tétraméthylammonium et éventuellement des cations Na$^-$, cette offrétite, ayant un rapport molaire SiO$_2$/Al$_2$O$_3$ compris entre 4 et 10, étant soumise d'abord à au moins un échange cationique précédé éventuellement d'une calcination sous air, en vue d'abaisser la teneur en potassium entre environ 1,5 et 3 % poids, puis étant soumise ensuite à au moins un cycle comportant au moins deux étapes, à savoir (a) au moins un traitement thermique entre 400 et 900 °C et (b) au moins un traitement en phase liquide, ce traitement en phase liquide étant un traitement choisi dans le groupe constitué par un échange cationique (effectué dans au moins une solution d'un sel d'ammonium ionisable, à une température comprise entre 0 et 150 °C) et une attaque acide (effectuée en présence d'un acide minéral ou un acide organique à une température comprise entre 0 et 150 °C), l'étape (b) comportant toujours au moins un échange cationique et au moins une attaque acide, cet échange et cette attaque étant réalisés soit dans un même cycle s'il n'y a qu'un seul cycle soit s'il y a plusieurs cycles dans un même cycle ou dans deux cycles différents ou à la fois dans un même cycle et dans deux cycles différents.

5. Procédé d'obtention d'une offrétite modifiée selon la revendication 4 dans lequel au moins un traitement thermique de l'étape (a) est une calcination en présence de vapeur d'eau ou une calcination en atmosphère statique (self-steaming).

6. Procédé selon l'une des revendications 4 et 5, dans lequel on effectue deux cycles, le premier cycle comportant une calcination et au moins un échange cationique, le deuxième cycle comportant une calcination, au moins un échange cationique et au moins une attaque acide.

7. Procédé selon la revendication 6 comportant un troisième cycle lui-même comportant au moins une calcination, au moins un échange cationique et au moins une attaque acide.

8. Procédé selon l'une des revendications 4 à 7, dans lequel on effectue au moins deux cycles, chaque cycle comportant la séquence self-steaming, échange cationique et attaque acide.

9. Offrétites selon l'une des revendications 1 à 3 caractérisées en ce qu'elles sont obtenues selon l'une des revendications 4 à 8.

10. Utilisation d'au moins une offrétite selon l'une des revendications 1 à 3 ou 9 comme catalyseur ou constituant d'un catalyseur dans les réactions de conversion en hydrocarbures du méthanol ou du diméthyléther ou d'un mélange de méthanol et de diméthyléther.

**Claims**

1. Offretite whose characteristics are :

— $SiO_2/Al_2O_3$ molar ratio higher than 30
— $\vec{a}$ and $\vec{c}$ crystalline parameters respectively ranging from 1.285 to 1.310 nm for $\vec{a}$ and from 0.748 to 0.755 nm for $\vec{c}$
— potassium content lower than 1 % by weight
— benzene adsorption capacity at 25 °C, under a partial pressure of 70 torrs (70 × 133.32 Pa), higher than 5 % by weight,
— a secondary microporosity ranging from 3 to 5 nm and corresponding to about 5-50 % of the total pore volume of the zeolite of offretite structure.

2. Offretite according to claim 1, whose crystalline parameters $\vec{a}$ and $\vec{c}$ respectively range from 1.285 to 1.305 nm for $\vec{a}$ and from 0.748 to 0.755 nm for $\vec{c}$.

3. Offretite according to claim 2, having the following characteristics :

— $SiO_2/Al_2O_3$ molar ratio higher than 50,
— $\vec{a}$ and $\vec{c}$ crystalline parameters ranging from 1.290 to 1.300 nm for $\vec{a}$ and from 0.748 to 0.755 nm for $\vec{c}$,
— potassium content lower than 0.5 % by weight,
— benzene adsorption capacity, at 25 °C and under a pressure of 70 torrs (133.32 × 70 Pa), higher than 6 % by weight,
— existence of a secondary microporosity, ranging from 3 to 5 nm and corresponding to about 5-50 % of the total pore volume of the zeolite of offretite structure.

4. A process for manufacturing a modified offretite according to claim 1, said modified offretite being prepared from an offretite comprising potassium $K^-$ cations, tetramethylammonium ions and optionally $Na^-$ cations, said offretite having a $SiO_2/Al_2O_3$ molar ratio from 4 to 10 being first subjected to at least one cation exchange, optionally preceded by a roasting in air, in order to decrease its potassium content to about 1.5-3 % by weight, then subjected to at least one cycle comprising at least two stages, respectively (a) at least one thermal treatment between 400 and 900 °C and (b) at least one liquid phase treatment, said liquid phase treatment being a treatment selected from the group consisting of a cation exchange (conducted in at least one solution of an ionizable ammonium salt, at a temperature from 0 to 150 °C) and an acid etching (conducted in the presence of an inorganic acid or an organic acid at a temperature from 0 to 150 °C), the step (b) always containing at least one cationic exchange and at least one acid etching, said exchange and etching being carried out either in the same cycle (when there is only one cycle) or when there are several cycles in a same cycle or in two different cycles or at the same time in a same cycle and in two different cycles.

5. A process for manufacturing a modified offretite according to claim 4, wherein at least one thermal treatment of step (a) consists of a roasting in the presence of steam or a roasting in static atmosphere (self-steaming).

6. A process according to one of claims 4 or 5, comprising two cycles, the first cycle including a roasting and at least one cationic exchange, the second cycle comprising a roasting, at least one cationic exchange and at least one and acid etching.

17

7. A process according to claim 6, further comprising a third cycle including at least one roasting, at least one cation exchange and at least one acid etching.

8. A process according to one of claims 4 to 7, comprising at least two cycles, each cycle including the sequence of self-steaming, cation exchange and acid etching.

9. Offretites according to one of the claims 1 to 3 characterized in that they are obtained according to one of the claims 4 to 8.

10. The use of at least one offretite according to claims 1 to 3 or 9 as catalyst or as constituent of a catalyst in the reactions of conversion to hydrocarbons of methanol or dimethylether or of a methanol and dimethylether mixture.

**Patentansprüche**

1. Offretit mit folgenden Merkmalen :

— molares Verhältnis $SiO_2/Al_2O_3$ über 30

— Kristallparameter $\vec{a}$ und $\vec{c}$ jeweils zwischen 1,285 und 1,310 nm für $\vec{a}$ und zwischen 0,748 und 0,755 nm für $\vec{c}$

— Kaliumgehalt unterhalb 1 Gew.-%

— Adsorptionskapazität für Benzol bei 25 °C bei einem Partialdruck von 70 Torr (70 × 133,32 Pa) über 5 Gew.-%

— sekundäre Mikroporosität zwischen 3 und 5 nm und entsprechend bei etwa 5 bis 50 % des Gesamtporenvolumens des Zeoliths der Offretitstruktur.

2. Offretit nach Anspruch 1, dessen Kristallparameter $\vec{a}$ und $\vec{c}$ jeweils zwischen 1,285 und 1,305 nm für $\vec{a}$ und zwischen 0,748 und 0,755 nm für $\vec{c}$ liegen.

3. Offretit nach Anspruch 2, wobei dieser Offretit die folgenden Merkmale aufweist :

— molares Verhältnis $SiO_2/Al_2O_3$ über 50

— Kristallparameter $\vec{a}$ und $\vec{c}$ zwischen 1,290 und 1,300 nm für $\vec{a}$ und zwischen 0,748 und 0,755 nm für $\vec{c}$

— Kaliumgehalt unterhalb 0,5 Gew.%

— Adsorptionskapazität für Benzol bei 25 °C und einem Druck von 70 Torr (70 × 133,32 Pa) über 6 Gew.-%

— Vorliegen einer sekundären Mikroporosität zwischen 3 und 5 nm und entsprechend etwa 5 bis 50 % des Gesamtporenvolumens des Zeoliths der Offretitstruktur.

4. Verfahren zur Herstellung eines modifizierten Offretits gemäß Anspruch 1, dadurch gekennzeichnet, daß dieser modifizierter Offretit hergestellt wird ausgehend von einem Offretit, der Kaliumkationen $K^+$, Tetramethylammoniumionen und ggfs. Kationen $Na^+$ aufweist und dieser Offretit mit einem molaren Verhältnis $SiO_2/Al_2O_3$ zwischen 4 und 10 zuerst mindestens einem Kationenaustausch, ggfs. nach Calcinieren an Luft, unterworfen wird, um den Gehalt an Kalium um etwa 1,5 bis 3 Gew.-% zu erniedrigen und dann zumindest einem Zyklus unterworfen wird, der zumindest zwei Stufen aufweist, nämlich (a) zumindest einer thermischen Behandlung zwischen 400 und 900 °C und (b) zumindest einer Behandlung in flüssiger Phase, wo bei dieser Behandlung in flüssiger Phase eine Behandlung ist, die aus der Gruppe gewählt wird, die aus einem Kationenaustausch (bewirkt in zumindest einer Lösung eines ionisierbaren Ammoniumsalzes, bei einer Temperatur zwischen 0 und 150 °C) und einem Säureangriff (bewirkt in Gegenwart einer Mineralsäure oder einer organischen Säure bei einer Temperatur zwischen 0 und 150 °C) durchgeführt wird, wobei die Stufe (b) immer zumindest einen Kationenaustausch und zumindest einen Säureangriff umfaßt und dieser Austausch und dieser Säureangriff entweder im gleichen Zyklus, wenn es sich um einen einzigen Zyklus handelt, oder wenn es sich um mehrere Zyklen handelt, im gleichen Zyklus oder in zwei verschiedenen Zyklen oder einmal in einem gleichen Zyklus und dann in zwei verschiedenen Zyklen durchgeführt wird.

5. Verfahren zur Herstellung eines modifizierten Offretits nach Anspruch 4, dadurch gekennzeichnet, daß zumindest eine thermische Behandlung der Stufe (a) eine Calcinierung in Gegenwart von Wasserdampf oder eine Calcinierung in statischer Atmosphäre (im geschlossenen Gefäß unter eigenem Dampfdruck) umfaßt.

6. Verfahren nach einem der Ansprüche 4 und 5, dadurch gekennzeichnet, daß man zwei Zyklen durchführt, wobei der erste Zyklus eine Calcinierung von zumindest einem Kationenaustausch umfaßt und der zweite Zyklus eine Calcinierung, zumindest einen Kationenaustausch und zumindest einen Säureangriff umfaßt.

7. Verfahren nach Anspruch 6, dadurch gekennzeichnet, daß man einen dritten Zyklus durchführt, der zumindest eine Calcinierung, zumindest einen Kationenaustausch und zumindest einen Säureangriff umfaßt.

8. Verfahren nach einem der Ansprüche 4 bis 7, dadurch gekennzeichnet, daß man zumindest zwei Zyklen durchführt, wobei jeder Zyklus die Folge Erhitzen im geschlossenen Gefäß, Kationenaustausch und sauren Angriff umfaßt.

9. Offretite nach einem der Ansprüche 1 bis 3, dadurch gekennzeichnet, daß sie nach einem der Ansprüche 4 bis 8 erhalten sind.

10. Verwendung von zumindest einem Offretit nach einem der Ansprüche 1 bis 3 oder 9 als Katalysator oder Bestandteil eines Katalysators bei den Umwandlungsreaktionen von Methanol oder Dimethyläther oder einem Gemisch von Methanol und Dimethyläther zu Kohlenwasserstoffen.